# EUROPEAN PATENT APPLICATION

(11) **EP 1 491 893 A1**
(43) Date of publication of application: **29.12.2004**
(21) Application number: 03708627.9
(22) Date of filing: 14.03.2003
(51) Int. Cl.: G01N 33/545, G01N 33/53, G01N 33/547, C12N 15/09, C12M 1/00

(54) **SUPPORT FOR AMINATED SUBSTANCE FIXING**

(30) Priority: 15.03.2002 US 364477 P
(71) Applicant: Universal Bio Research Co., Ltd., Matsudo-shi, Chiba 271-0064 (JP)
(72) Inventor: Tajima, Hideji, Matsudo-shi, Chiba 271-0064 (JP); Stimpson, Donald I., Bio-Strand, Inc., Aurora, CO 800-7337 (US)
(74) Representative: Bohnenberger, Johannes, Dr.
(86) International application number: PCT/JP2003/003092
(87) International publication number: WO 2003/079019

(57) **Abstract**

An object of the present invention is to provide a carrier for fixing a material containing an amino group, which has a shape to be wound on a spindle member, and on the surface of which the material can be immobilized via covalent bonding; and in order to achieve this object, the carrier of the present invention is a carrier which has a shape to be wound on a spindle member, and has a carboxyl group or an amino group, or derivative groups thereof formed by cleaving an amide bond in the main chain of a polyamide on its surface.

## Description

### TECHNICAL FIELD

The present invention relates to a carrier for fixing a material containing an amino group, on the surface of which the material can be immobilized; a carrier on the surface of which a material containing an amino group has been immobilized; and a method of immobilizing a material containing an amino group on a carrier.

### BACKGROUND ART

Generally, when detecting and isolating a labeled nucleic acid, a probe comprising a base sequence complementary to that of the labeled nucleic acid is immobilized on a solid carrier (solid support). After the labeled nucleic acid and the probe immobilized on the solid carrier have been allowed to hybridize and materials other than the labeled nucleic acid have been removed by rinsing, etc., the labeled nucleic acid is detected and isolated.

One type of solid carrier with immobilized probes is a DNA array (DNA chip) wherein multiple probes are arrayed and immobilized on the surface of a solid carrier such as a glass slide. A DNA array is extremely useful in conducting parallel analysis of the expression, mutation and polymorphism of genes.

Well-known methods to produce a DNA array include, for example, directly synthesizing oligonucleotides used as the probe on the surface of the solid carrier, and immobilizing pre-prepared oligonucleotides (polynucleotides in some cases) as the probe on the surface of the solid carrier.

A representative example of the former method is to use a blocking group that is selectively removed by laser irradiation, to combine photolithography used in semiconductor manufacturing with solid phase synthesis technology, and to selectively synthesize the probe in specific regions of a matrix.

Moreover, representative examples of the latter are: (1) affixing cDNA and PCR products to the surface of a solid carrier that has been surface treated with positive poly-ions (polylysine, polyethylene imine, etc.), and utilizing DNA electrical charges to cause electrostatic bonding on the solid carrier; and (2) synthesizing oligonucleotides to which a reactive group is introduced, affixing the aforementioned oligonucleotide to the surface of a surface-treated solid carrier, and allowing covalent bonding on the surface of the solid carrier.

When producing a DNA array, the goal is to reliably and efficiently immobilize the probe on a solid carrier by selecting a suitable method corresponding to the type of nucleic acid used as the probe, and to the type of solid carrier.

### DISCLOSURE OF THE INVENTION

The general embodiment of a DNA array is a high density array of multiple types of DNA on the surface of a flat solid carrier.

Nonetheless, when making a high density array of multiple types of DNA on a flat surface, cross-contamination of different types of DNA is prone to occur. It appears that cross-contamination can be prevented by using small amounts of DNA at each site, but the responsiveness is diminished when using small amounts of DNA. Moreover, it is very costly to manufacture DNA arrays because it requires prodigious time and labor to make high density arrays of multiple types of DNA on a flat surface.

Therefore, in order to resolve these problems, the present inventors developed an integrated support comprising a filament-like, string-like or tape-like elongated foundation member and a support on which the aforementioned foundation member is wound, wherein various types of materials for detection having specific chemical structures are fixed longitudinally in lines and the various chemical structures are made to correspond with fixed positions thereof. Patent applications (WO 01/69249A1, WO 01/53831A1 and WO 02/63300A1) have already been filed.

When using the integrated technology developed by the present inventors, it is possible to produce a DNA array with DNA arrayed in high density at low cost while preventing cross-contamination by immobilizing the DNA on the filament-like, string-like or tape-like elongated solid carrier (primary integration), and then winding this on a spindle member (secondary integration). In order to realize this kind of DNA array, it is necessary to reliably and efficiently immobilize the DNA on the filament-like, string-like or tape-like elongated solid carrier.

Meanwhile, if a nucleotide having an amino group (for example, deoxyadenylic acid, deoxycytidylic acid, or deoxyguanylic acid) is comprised in DNA, then the DNA contains an amino group. Moreover, it is possible to introduce an amino group artificially into various types of chemical materials, especially DNA, by chemical modification.

Thus, a first object of the present invention is to provide a carrier for fixing a material containing an amino group, which has a shape to be wound on a spindle member, and on the surface of which the material can be immobilized via covalent bond.

Moreover, a second object of the present invention is to provide a carrier, which has a shape to be wound on a spindle member, and on the surface of which a material containing an amino group has been immobilized via covalent bond.

Further, a third object of the present invention is to provide a method of immobilizing a material on the surface of a carrier having a shape to be wound on a spindle member.

In order to achieve the aforementioned object, the present invention provides a carrier for fixing a material containing an amino group, a carrier on the surface of which a material containing an amino group has been immobilized, and a method of immobilizing a material containing an amino group.
(1) A carrier for fixing a material containing an amino group, which has a shape to be wound on a spindle member, and has a carboxyl group or an amino group, or a derivative group thereof formed by cleaving an amide bond in the main chain of a polyamide on its surface.
(2) The carrier described in the aforementioned (1), obtained by hydrolyzing a crude material member which has a shape to be wound on a spindle member, and has a polyamide exposed on its surface, to cleave an amide bond in the main chain of the polyamide.
(3) The carrier described in the aforementioned (2), wherein the crude material member is a polyamide fiber or an aggregate thereof.
(4) The carrier described in any of the aforementioned (1) to (3), wherein the shape of the carrier is filament-like, string-like or tape-like.
(5) The carrier described in any of the aforementioned (1) to (3), wherein the amino group formed by cleaving the amide bond in the main chain of the polyamide is converted to a carboxyl group or a derivative group thereof by reacting the amino group with a cyclic acid anhydride.
(6) A carrier on the surface of which a material containing an amino group has been immobilized, obtained by reacting a carboxyl group or a derivative group thereof on the surface of the carrier described in any of the aforementioned (1) to (3) with the amino group of the material, to immobilize the material on the surface of the carrier via an amide bond.
(7) A method of immobilizing a material containing an amino group on a carrier which has a shape to be wound on a spindle member, and has a polyamide exposed on its surface; the method comprising:
   (a) a process to produce a carboxyl group or an amino group, or a derivative group thereof on the surface of the carrier by hydrolyzing the carrier to cleave an amide bond in the main chain of the polyamide; and
   (b) a process to immobilize the material on the surface of the carrier via an amide bond by reacting the carboxyl group or the derivative group thereof on the surface of the carrier with the amino group of the material.
(8) The method described in the aforementioned (7), wherein the amino group on the surface of the carrier is converted to a carboxyl group or a derivative group thereof by reacting the amino group with a cyclic acid anhydride, prior to the process (b).

### BRIEF DESCRIPTION OF THE DRAWINGS

Figure 1 is a diagram indicating the changes of absorbance after immobilizing a probe on nylon threads hydrolyzed with 2 N hydrochloric acid for a specified time at 40° C, and then allowing this to react with labeled nucleic acid;
Figure 2 is a diagram indicating the changes of absorbance after immobilizing probes of various concentrations on nylon threads hydrolyzed with 2 N hydrochloric acid for one hour at 40° C, and then allowing this to react with various concentrations of labeled nucleic acid;
Figure 3 is a diagram indicating the results of measuring coloration after immobilizing a probe on nylon threads hydrolyzed with various concentrations of hydrochloric acid for one hour at 40° C, and then allowing this to react with labeled nucleic acid;
Figures 4 (a) and (b) are diagrams indicating the results of measuring coloration after immobilizing a probe on nylon threads hydrolyzed and then treated with succinic acid anhydride, and then allowing this to react with labeled nucleic acid. Figures 4 (c) and (d) are diagrams indicating the results of measuring coloration after immobilizing a probe on nylon threads hydrolyzed without treating with succinic acid anhydride, and then allowing this to react with the labeled nucleic acid;
Figures 5 (a) and (b) are diagrams indicating the results of measuring coloration after immobilizing a probe on nylon threads hydrolyzed and then treated with succinic acid anhydride, and then allowing this to react with various concentrations of labeled nucleic acid;
Figure 6 is a diagram indicating the results of measuring fluorescence intensity after immobilizing a probe respectively on a cloudy, semi-transparent nylon thread obtained by hydrolysis treatment with 4 N hydrochloric acid, and on a transparent nylon thread obtained by hydrolysis treatment with 2.5 N hydrochloric acid for one hour at 37° C, and then allowing this to react with various concentrations of labeled nucleic acid; and
Figure 7 is a partial cross-sectional diagram indicating one embodiment of an apparatus used when conducting hydrolysis treatment of the crude material member having an elongated shape such as string-like or tape-like shape.

### BEST MODE FOR CARRYING OUT THE INVENTION

The present invention will be explained in detail below.

The carrier of the present invention is a carrier for fixing a material containing an amino group.

The carrier of the present invention has a shape to be wound on a spindle member. As long as the spindle member can be the center of a wound object, the shape and the structure of the spindle member are not particularly limited. Examples of the spindle member include rod, column, cylinder, polygonal column, and polygonal cylinder. Examples of the shape to be wound on the spindle member include, but are not limited to, elongated shape such as filament-like, string-like, tape-like shape and so on.

The carrier of the present invention has a polyamide as a necessary component. As long as the polyamide is a polymer having amide bonds in the main chain thereof, the type of polyamide is not particularly limited. Examples of the polyamide include a polymer obtained by ring-opening polymerization of lactam (for example, nylon 6, nylon 12, etc.), a polymer obtained by polycondensation of amino carboxylic acid (for example, nylon 11, etc.) and a polymer obtained by polycondensation of diamine and dicarboxylic acid (for example, nylon 66, nylon 6, 10, etc.).

The carrier of the present invention may consist of polyamide, or may comprise a component other than polyamide. Because the carrier of the present invention is mainly used in an aqueous medium, the carrier is preferably insoluble in water, and resistant to swelling. Moreover, the carrier of the present invention preferably has flexibility to be wound on a spindle member. An example of component other than polyamide of the carrier include water-insoluble polymer. Examples of water-insoluble polymer include a polymer obtained by polymerizing one or more kinds of vinyl-based monomers such as aromatic vinyl compounds, esters or amides of α,β-unsaturated carboxylic acid, α,β-unsaturated nitrile compounds, halogenated vinyl compounds, conjugated diene compounds, and vinyl esters of lower fatty acid (for example, styrene, chlorostyrene, chloromethyl styrene, α-methyl styrene, divinyl benzene, sodium styrene sulfonate, (meth)acrylate, methyl (meth)acrylate, ethyl (meth)acrylate, n-butyl (meth)acrylate, 2-hydroxyethyl (meth)acrylate, (meth)acrylate polyoxyethylene, glycidyl (meth)acrylate, ethylene glycol di-(meth)acrylate, tribromophenyl (meth)acrylate, tribromo propyl acrylate, (meth)acrylonitrile, (meth)acrolein, (meth)acrylamide, methylene bis (meth)acrylamide, butadiene, isoprene, vinyl acetate, vinylpyridine, N-vinylpyrrolidone, vinyl chloride, vinyl bromide); cross-linked polysaccharides such as agarose, dextran, cellulose, and carboxymethyl cellulose; and cross-linked protein such as gelatin, methylated albumin, collagen, and casein. Other examples of component of the carrier include glass, ceramic, and silicon.

Among polyamides comprised in the carrier of the present invention, part or all of the polyamides existing on the surface of the carrier are treated by hydrolysis (after hydrolysis treatment, a suitable derivatization treatment may be conducted as necessary). Specifically, a carboxyl group or an amino group, or a derivative group thereof formed by cleaving an amide bond in the main chain of a polyamide is present on the surface of the carrier of the present invention.

The carrier of the present invention may, for example, be obtained by hydrolyzing a crude material member which has a shape to be wound on a spindle member and which has a polyamide exposed on its surface, to cleave an amide bond in the main chain of the polyamide.

A polyamide fiber or an aggregate thereof (for example, a plied polyamide fiber), and a fiber comprising the aforementioned water-insoluble polymer as a main component, or an aggregate thereof that have been surface coated with polyamide may be used as the crude material member.

The hydrolysis treatment of the crude material member may be conducted by conventional methods in the presence of an acid or a base.

The acid used for hydrolysis treatment is not particularly limited, and examples of the acid include inorganic acid such as hydrochloric acid, hydrobromic acid, hydrofluoric acid and sulfuric acid; and organic acid such as formic acid, acetic acid, and propionic acid. Preferably, the acid is hydrochloric acid. The amount of acid used is normally 0.5 to 6 N, and preferably, 2 to 4 N.

Moreover, the base used for hydrolysis treatment is not particularly limited, and examples of the base include inorganic base such as hydroxide, carbonate or bicarbonate of alkali metals (for example, lithium, sodium, or potassium, etc.) or alkali earth metals (for example, calcium, or magnesium, etc.); and organic base such as trialkylamine (for example, trimethylamine, triethylamine, etc.). Preferably, the base is sodium hydroxide or potassium hydroxide. The amount of base used is normally 0.1 to 4 N, and preferably, 1 to 2 N.

Other than water, solvents that do not inhibit hydrolysis reaction, for example, alcohols solvent such as methanol and ethanol, tetrahydrofuran (THF), and dioxane may be contained in the medium used for hydrolysis treatment.

When conducing hydrolysis treatment using 2 to 4 N hydrochloric acid, normally, the reaction temperature is 5 to 60° C, and preferably room temperature to 40° C; and the reaction time is normally 0.05 to 24 hours, preferably 0.1 to 2 hours. Most preferably, hydrolysis treatment is conducted at 40° C for about one hour using 2.5 N hydrochloric acid. If the hydrolysis treatment is excessive, the strength of the carrier of the present invention will be reduced, and if the hydrolysis treatment is insufficient, the amount of carboxyl groups and amino groups formed by cleaving the amide bonds will be insufficient. However, if the aforementioned reaction conditions are used, a sufficient amount of carboxyl groups and amino groups can be formed on the surface of the carrier while maintaining the strength thereof.

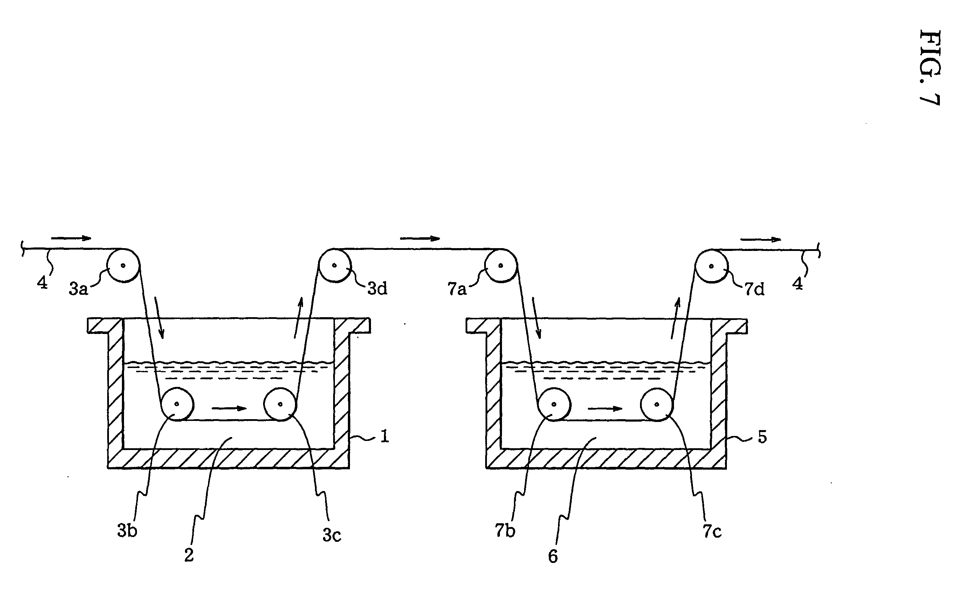

If using a crude material member having a shape to be wound, as represented by an elongated shape such as filament-like, string-like or tape-like shape, the crude material member can be efficiently hydrolyzed by continuously passing the crude material member through a solution containing an acid or a base (specifically, by continuously immersing the crude material member in the aforementioned solution). For example, as indicated in Figure 7, a crude material member 4 having an elongated shape such as filament-like, string-like or tape-like shape can be continuously hydrolyzed by continuously immersing in a solution 2 containing an acid or a base in a container 1 by using four rollers 3a, 3b, 3c, and 3d. The solution 2 adheres to the crude material member 4 that has passed through the solution 2 in the container 1, and there is concern that, left as is, hydrolysis may advance beyond what is necessary. Therefore, as indicated in Figure 7, the solution 2 adhering to the crude material member 4 can be rinsed off and further hydrolysis of the crude material member 4 can be prevented by continuously immersing the crude material member 4 that has passed through the solution 2 in rinse water 6 in a container 5 by using four rollers 7a, 7b, 7c, and 7d. The amide bond in the main chain of the polyamide on the surface of the crude material member is cleaved into a carboxyl group and an amino group by subjecting the crude material member to hydrolysis treatment in the aforementioned manner. After hydrolysis treatment, the carboxyl group or the amino group may be modified into a suitable derivative group by subjecting the carboxyl group or the amino group to suitable derivatization treatment.

The derivatization treatment of the carboxyl group formed by cleaving the amide bond as well as the type of the derivative group formed by the derivatization treatment are not particularly limited as long as the derivative group has reactivity to an amino group. Specifically, the carboxyl group on the surface of the carrier of the present invention has the role of reacting with an amino group of a material to be immobilized, and immobilizing the material to the surface of the carrier of the present invention via an amide bond. Therefore, the derivative group of the carboxyl group is not particularly limited as long as the derivative group can react with an amino group to form an amide bond. Examples of derivatization treatment and derivative group include a derivative group - COX (X expresses halogen.) formed by halogenation of carboxyl group, and a derivative group - CONH-(CH₂)ₙ-COOH formed by allowing a carboxyl group to react with amino carboxylic acid NH₂-(CH₂)ₙ-COOH (n is an optional natural number, and is normally a natural number from 2 to 12.).

Moreover, the derivatization treatment of the amino group formed by cleaving the amide bond as well as the type of derivative group formed by the derivatization treatment are not particularly limited as long as the derivative group do not have reactivity to a carboxyl group. Specifically, immobilization of a material containing an amino group to the surface of the carrier of the present invention is conducted by activating the carboxyl group on the surface of the carrier of the present invention, and allowing the activated carboxyl group to react with the amino group of the material to be immobilized. However, if an amino group formed by cleaving the amide bond on the surface of the carrier of the present invention is present at this time, there is concern that this amino group will react with the activated carboxyl group, and the immobilization of the material containing an amino group onto the surface of the carrier of the present invention will be prevented. Consequently, the derivative group of the amino group is not particularly limited as long as the derivative group is not reactive with a carboxyl group. Examples of derivatization treatment of a amino group include deactivation of the amino group, and modification of the amino group into a carboxyl group or a derivative group thereof. An example of deactivation of the amino group includes acetylization of the amino group, and a derivative group -NHCOCH₃ is formed by the acetylization. Moreover, the conversion of an amino group into a carboxyl group can be conducted by allowing a cyclic acid anhydride to react with the amino group, and a carboxyl group thus formed can be modified into a derivative group by conducting the derivatization treatment described above. By converting an amino group into a carboxyl group or a derivative group thereof, it is possible to immobilize more materials containing an amino group on the surface of the carrier of the present invention.

Examples of cyclic acid anhydride used when converting an amino group into a carboxyl group include malonic acid anhydride, oxalic acid anhydride, succinic acid anhydride, glutaric acid anhydride, and adipic acid anhydride. Preferably, a cyclic acid anhydride is succinic acid anhydride. Derivative groups -NHCO-CH₂-COOH, -NHCO-COOH, -NHCO-(CH₂)₂-COOH, -NHCO-(CH₂)₃-COOH, -NHCO-(CH₂)₄-COOH are formed respectively by reacting an amino group with malonic acid anhydride, oxalic acid anhydride, succinic acid anhydride, glutaric acid anhydride, and adipic acid anhydride.

The conversion of an amino group to a carboxyl group can, for example, be conducted by treating a carrier that has an amino group on its surface with a buffer solution (for example, a calcium phosphate buffer solution (pH 6.0)) containing 0.8 M succinic acid at room temperature over night. The conversion can be confirmed using a ninhydrin reagent. (A blue color will appear if an unreacted amino group is present.)

The material containing an amino group can be immobilized via an amide bond on the surface of the carrier of the present invention by allowing a carboxyl group or a derivative thereof on the surface of the carrier of the present invention to react with an amino group of the material to be immobilized.

The type of material containing an amino group to be immobilized on the carrier of the present invention is not particularly limited as long as it contains an amino group, and examples of material containing an amino group include nucleic acid and protein containing an amino group.

If a nucleotide containing an amino group (for example, adenylic acid, cytidylic acid, guanylic acid, deoxyadenylic acid, deoxycytidylic acid, and deoxyguanylic acid) is comprised in a nucleic acid, the nucleic acid contains an amino group. Therefore, it is not particularly necessary to artificially introduce an amino group to the nucleic acid (of course, an amino group may be artificially introduced to the nucleic acid), but if this kind of nucleotide is not comprised, then an amino group may be artificially introduced to the nucleic acid. Artificial introduction of an amino group may be conducted by amination via chemical modification (for example, introduction of an amino alkyl group), or by an addition reaction of an aminated linker using DNA ligase. From the perspective of improving the efficiency of hybridization with the labeled nucleic acid, the nucleic acid immobilized on the carrier of the present invention preferably has an amino group on a 5' terminal or a 3' terminal, or neighborhood thereof, and is immobilized on the surface of the carrier of the present invention via the amino group. Further, the nucleic acid may be either an oligonucleotide or a polynucleotide, and nucleotides comprised in the nucleic acid may be either deoxyribonucleotides or ribonucleotides. Specifically, the nucleic acid may be either DNA or RNA. Moreover, the base sequence and the base length of the nucleic acid are not particularly limited, and the base sequence and the base length may be selected so that the nucleic acid can be hybridized with the target nucleic acid. The nucleic acid may be immobilized in a double chain or single chain state.

If the N terminal of protein is a free amino group, the protein contains an amino group, and therefore it is not particularly necessary to artificially introduce an amino group to the protein (of course, an amino group may be artificially introduced to the protein), but if the N terminal is not a free amino group, then an amino group may be artificially introduced by amination via chemical modification.

Both a carboxyl group or a derivative thereof formed by cleaving an amide bond and a carboxyl group or a derivative thereof formed by converting an amino group formed by cleaving an amide bond are included in a carboxyl group or a derivative thereof on the surface of the carrier that reacts with an amino group of the material to be immobilized. If there is an amino group formed by cleaving an amide bond on the surface of the carrier, this amino group reacts with the activated carboxyl group, and inhibits immobilization of the material containing an amino group on the surface of the carrier. Therefore, when immobilizing the material containing an amino group on the surface of the carrier, it is preferable to convert an amino group on the surface into a carboxyl groups or a derivative group thereof. By doing this, not only is there no inhibition of immobilization of the material containing an amino group onto the surface of the carrier, but it is also possible to immobilize more materials containing an amino group onto the surface of the carrier. Moreover, when immobilizing a material containing an amino group on the surface of the carrier, an amino group on the surface of the carrier may be made inactive.

When allowing a carboxyl group or a derivative group thereof on the surface of the carrier of the present invention to react with an amino group of the material to be immobilized, the carboxyl group or the derivative group thereof is activated. This kind of activation may be conducted by using, for example, carbodiimides such as 1-ethyl-3-(3-dimethylaminopropyl)carbodiimide (EDAC); hydroxyimides such as hydroxysuccinimide, or hydroxyphthalimide; chlorides such as thionyl chloride, or oxalyl chloride; or aqueous dehydration condensing agents such as Woodward's reagent K, or N-ethoxycarbonyl-2-ethoxy-1,2-dihydroquinoline (EEDQ).

Specifically, the reaction of a carboxyl group or a derivative group thereof on the surface of the carrier of the present invention with an amino group of the material to be immobilized may be conducted in the following manner. The carboxyl group or the derivative group thereof on the surface of the carrier is treated with an aqueous solution of 20% 1-ethyl-3-(3-dimethylaminopropyl)carbodiimide for about five minutes, and the carboxyl group or the derivative group thereof is activated. Then, the excess reagent is removed by rinsing with water or buffer solution. Next, this is processed for about 15 minutes after adding a buffer solution (for example, sodium bicarbonate buffer (pH 8.4)) containing the material to be immobilized, and the activated carboxyl group is allowed to react with the amino group of the material to be immobilized. Next, the remaining activated carboxyl group is blocked by adding PBS buffer containing 1% casein (or 5% SDS-1xSSC, or 1xDenhard solution-2xSSC).

A carrier on the surface of which the material containing an amino group is immobilized, is obtained as indicated above. In this kind of carrier for fixing a material containing an amino group, the material containing an amino group is immobilized on the surface of the carrier via an amide bond (covalent bond), and therefore, the material containing an amino group will not fall off the surface of the carrier. Consequently, it is possible to more easily and efficiently detect, isolate and screen for labeled nucleic acid and labeled protein by utilizing the DNA array or protein array produced by immobilizing the material containing an amino group such as nucleic acid and protein onto the surface of the carrier, and winding this on a spindle member.

A more detailed explanation of the present invention will be given using examples.

### [Example 1]

Nylon thread (mean diameter 100µm) composed of nylon 6 was subjected to hydrolysis treatment at 40° C, for the specified time in the presence of 2 N hydrochloric acid, and the amide bonds on the surface of the nylon thread were cleaved into carboxyl groups and amino groups. The hydrolysis times were 0 minutes, 30 minutes, 60 minutes, 90 minutes, and 120 minutes, and these were taken as groups I to V respectively.

After rinsing the nylon thread of groups I to V three times in water, the nylon thread was treated with 20% EDAC (1-ethyl-3-(3-dimethylaminopropyl)carbodiimide) at room temperature for 5 minutes, and the carboxyl groups on the surface of the nylon thread were activated. A 0.5 M sodium hydrogen carbonate aqueous solution (pH 8.5) containing 200 mM probe (aminated DNA wherein amino linker was introduced on the 5' terminal) was immediately applied to this nylon thread, and allowed to react for 15 minutes. The base sequence of the probe was 5'-agtggaggtcaacga-3'.

PBS containing 1% BSA was applied to the nylon thread of groups I to V that had reacted with the probe, the remaining activated carboxyl groups were blocked, and the nylon thread was stored at 4° C until use.

The nylon thread of groups I to V that had reacted with the probe was allowed to react with 20 µL of 500 nM labeled nucleic acid (biotinylated DNA) solution for 15 minutes. The base sequence of the labeled nucleic acid was 5'-tcgttgacctccact-3'.

Next, after allowing the nylon thread of groups I to V to react for 15 minutes with a 100 times dilution of avidinated alkali phosphatase solution, the nylon thread was rinsed 5 times in PBS containing 1% BSA, and 100 µL p-nitrophenylphosphate (p-NPP) solution, which is a substrate of an alkali phosphatase, was applied. After incubating for one hour at 37° C, 0.6 mL water was applied, and the absorbance at 405 nm was measured.

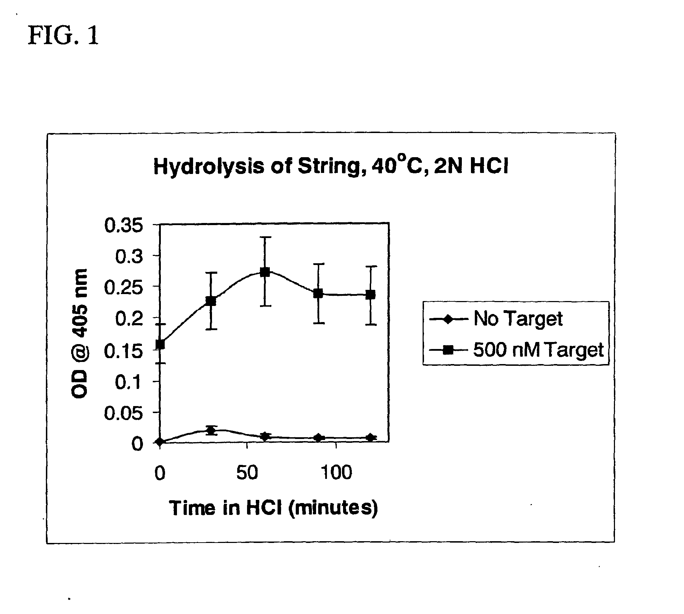

The results are indicated in Figure 1.

As indicated in Figure 1, in the presence of labeled nucleic acid, the absorbance increased in conjunction with the time of hydrolysis treatment, and a plateau was reached after the hydrolysis treatment time exceeded 60 minutes. From these results, it could be confirmed that the carboxyl groups on the surface of the nylon thread formed by cleaving amide bonds had reacted with the amino groups of the probe, and that the probe was immobilized on the surface of the nylon thread via amide bonding. Moreover, it was clarified that a suitable time for hydrolysis treatment of the nylon thread was about 60 minutes in 2 N hydrochloric acid at 40° C.

### [Example 2]

The 60 minutes reaction time used in Example 1 was specified, the concentrations of the probe and the labeled nucleic acids were varied, and the absorbance was measured in the same manner as in Example 1.

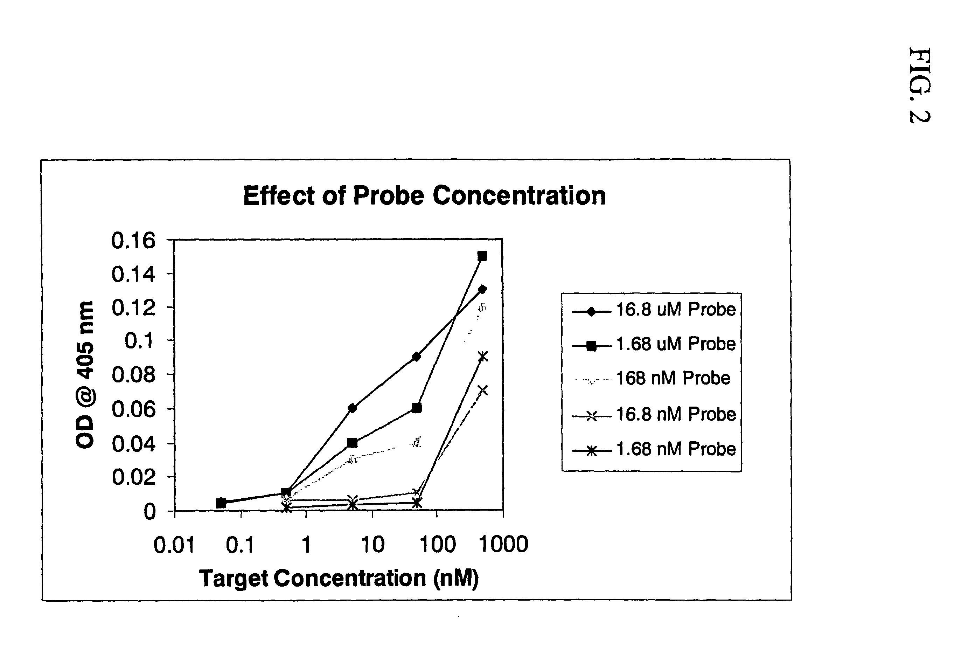

The results are indicated in Figure 2.

As indicated in Figure 2, the concentration of the labeled nucleic acids to react with the probe increased in conjunction with the increase of the concentration of the probe. From these results, it could be confirmed that the labeled nucleic acids are not non-specifically adsorbed to the surface of the nylon thread, but react (hybridize) with the probe immobilized on the nylon thread. Specifically, it could be confirmed that the labeled nucleic acid could be isolated and detected using the probe immobilized on the surface of the nylon thread.

### [Example 3]

The 60 minutes reaction time used in Example 1 was specified, and the concentrations of hydrochloric acid were varied at 0 N, 1 N, 2 N, 2.5 N, and 3N. After using BCIP/NBT (5-bromo-4-chloro-3-indolyl phosphate/nitroblue tetrazolium) as a substrate of alkali phosphatase, and incubating for 30 minutes at 37° C, the degree of coloration produced on the specified locations on the nylon threads was scored using detection image software (name of software: Scion Image; vendor: Scion Corporation).

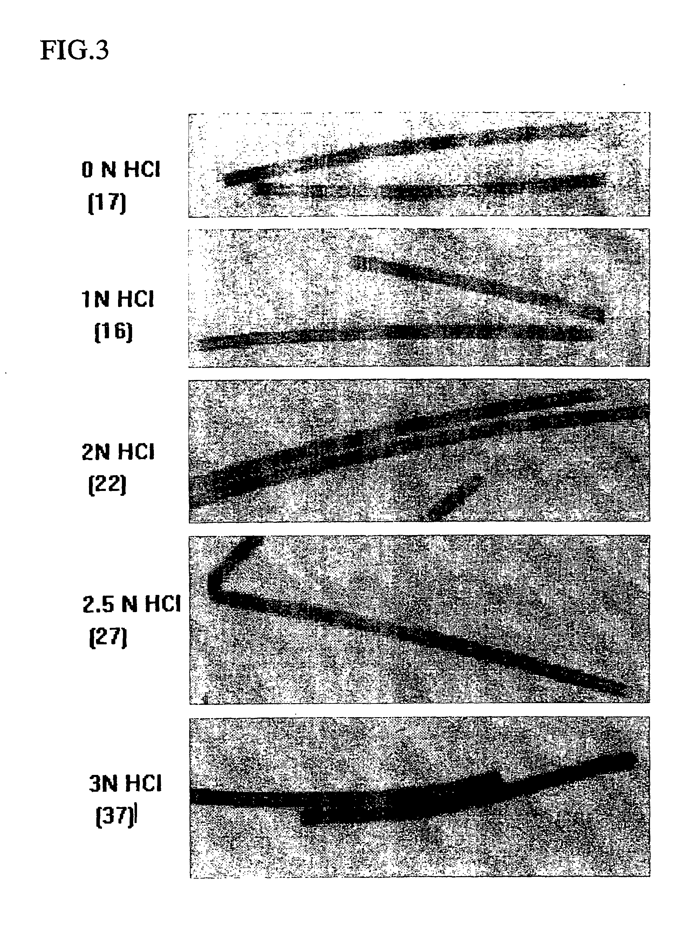

The results are indicated in Figure 3. The numeric values in the brackets in Figure 3 are the scores obtained by the aforementioned software processing, and the higher score indicates darker coloration. (Specifically, the higher the score, the more labeled nucleic acids had bonded to the nylon thread.)

As indicated in Figure 3, sufficient coloration was obtained with a hydrochloric acid concentration of 2 N or greater. From these results, it was clarified that a hydrochloric acid concentration of 2 N or greater is suitable for use in the hydrolysis treatment of the nylon thread. However, if the concentration of hydrochloric acid exceeds 2.5 N, the strength of the nylon thread decreases, and therefore, it appears that a hydrochloric acid concentration of 2.5 N is suitable. Combined with the results of Example 1, it appears that conducting the hydrolysis treatment of nylon thread at 40° C for about 1 hour using 2.5 N hydrochloric acid is suitable.

### [Example 4]

Using 2.5 N hydrochloric acid, hydrolysis treatment of the same nylon thread as in Example 1 was conducted at 37° C for 1 hour. The amino groups on the surface of the nylon thread were converted into carboxyl groups by immersing the hydrolysis-treated nylon thread over night in 0.5 M sodium phosphate aqueous solution (pH 6.0) containing 0.83 M succinic acid anhydride.

Thereafter, the nylon thread was rinsed three times in water, treated with 20% EDAC (1-ethyl-3-(3-dimethylaminopropyl)carbodiimide) at room temperature for 6 minutes, and the carboxyl groups on the surface of the nylon thread were activated. Approximately 0.05 µL of sodium hydrogen carbonate aqueous solution (pH 8.0) containing 166 µM probe (aminated DNA wherein amino linker was introduced on the 5'terminal) was spotted onto this nylon thread, and dried for 5 minutes. The base sequence of the probe was 5'-agtggaggtcaacga-3'.

PBS containing 1% BSA was applied to the nylon thread that had reacted with the probe, the remaining activated carboxyl groups were blocked, and the nylon thread was stored at 4° C until use.

The nylon thread that had reacted with the probe was allowed to react with 20 µL of 500 nM labeled nucleic acid (biotinylated DNA) solution for 15 minutes, and was then allowed to react for 15 minutes with a 100 times dilution of avidinated alkali phosphatase solution. The base sequence of the labeled nucleic acid was 5'-tcgttgacctccact-3'.

Next, the nylon thread was rinsed 5 times in PBS containing 1% BSA, and after using BCIP/NBT (5-bromo-4-chloro-3-indolyl phosphate/nitroblue tetrazolium) as a substrate of alkali phosphatase and incubating for 30 minutes at 37° C, the degree of coloration produced on the specified locations of the nylon threads was scored using detection image software (name of software: Scion Image; vendor: Scion Corporation).

The same procedures and measurements as above were conducted except for treatment with succinic acid anhydride, and the scores obtained from the aforementioned software processing were compared for when succinic acid anhydride treatment was conducted, and when not.

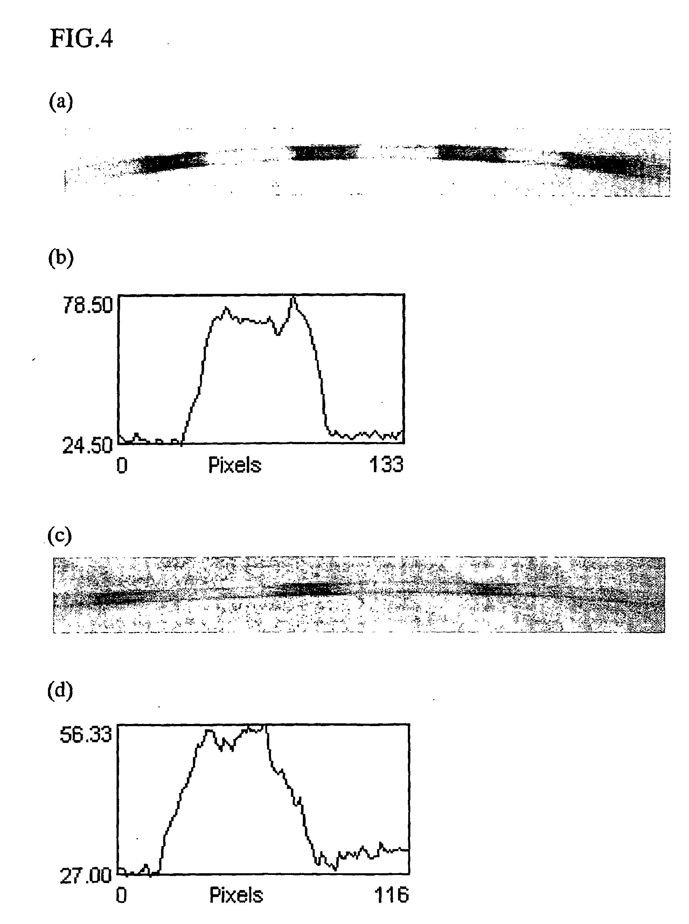

The results are indicated in Figure 4. In Figure 4, (a) indicates the state of coloration of the nylon thread treated with succinic acid anhydride; and (b) indicates the scores when scanning one spot (the second spot from the left in (a)) of the nylon thread from left to right. Moreover, (c) indicates the state of coloration of the nylon thread not treated with succinic acid anhydride; and (d) indicates the scores when scanning one spot (the second spot from the left in (c)) of the nylon thread from left to right.

As indicated in Figure 4, the scores for the nylon thread treated with succinic acid anhydride were greater (specifically, the color was darker) than those of the untreated nylon thread. From these results, it was clarified that it was possible to immobilize a greater amount of probe (aminated DNA) by converting the amino groups on the surface of the nylon thread formed by cleaving the amide bonds into carboxyl groups by treating with succinic acid anhydride.

### [Example 5]

The nylon thread which had treated with succinic acid anhydride and the probes had immobilized on in Example 4 was allowed to react with labeled nucleic acid of various concentrations (69 nM, 138 nM, 275 nM, 550 nM, and 1.1 µM). The degree of coloration produced on the specified locations of the nylon threads was scored using detection image software (name of software: Scion Image; vendor: Scion Corporation).

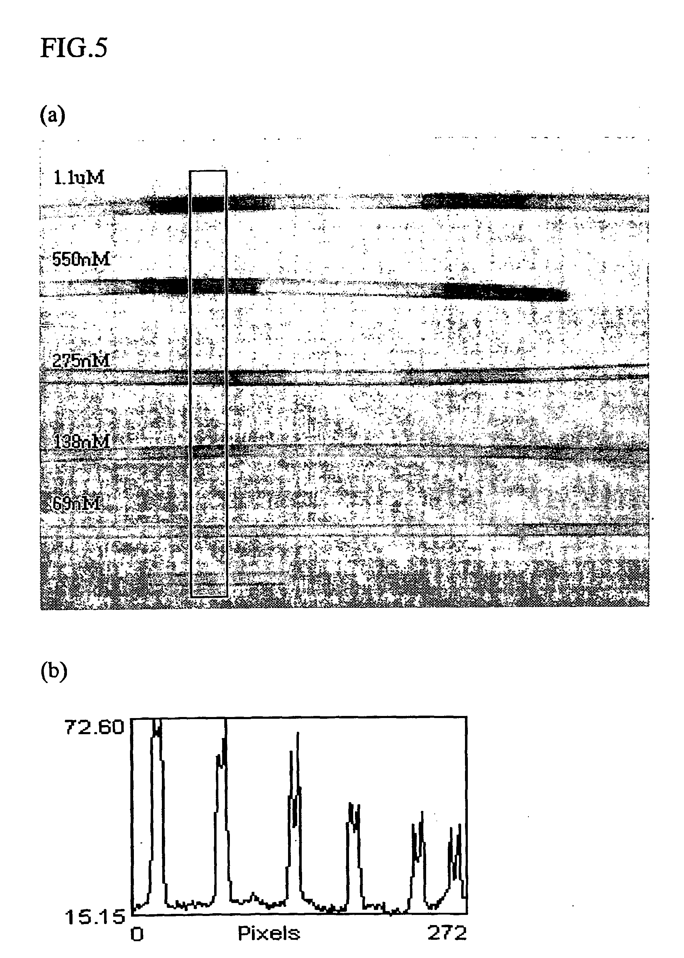

The results are indicated in Figure 5. In Figure 5, (a) indicates the state of coloration of the nylon threads allowed to react with various concentrations of labeled nucleic acid; and (b) indicates the scores when scanning one spot (the spots enclosed by the rectangle in (a)) of the nylon threads. The peaks in Figure 5(b) are the scores when allowing to react with labeled nucleic acid in concentrations of 1.1 µM, 550 nM, 275 nM, 138 nM, and 69 nM in order from the left. The lowest nylon thread in Figure 5(a) is the part between the spots (specifically, a part on which no probe was immobilized) of the nylon thread allowed to react with 69 nM labeled nucleic acid; and the peak on the furthest right of Figure 5(b) is the score of that part.

As indicated in Figure 5, the scores increased in conjunction with the increase of the concentration of the labeled nucleic acid. From these results, it could be confirmed that the labeled nucleic acids are not non-specifically adsorbed to the surface of the nylon thread, but react (hybridize) with the probe immobilized on the nylon thread. Specifically, it could be confirmed that the labeled nucleic acid could be isolated and detected using the probe immobilized on the surface of the nylon thread.

### [Example 6]

A probe was immobilized in the same manner as in Example 4 respectively onto a cloudy, semitransparent nylon thread obtained by hydrolysis treatment with 4 N hydrochloric acid, and onto a transparent nylon thread obtained by hydrolysis treatment with 2.5 N hydrochloric acid at 37° C for one hour. After hybridizing labeled nucleic acid (DNA labeled with Cy5) of various concentrations (11 nM, 114 nM, 228 nM) for 10 minutes in the presence of 2xSSC, 5% SDS, the fluorescent intensity was detected.

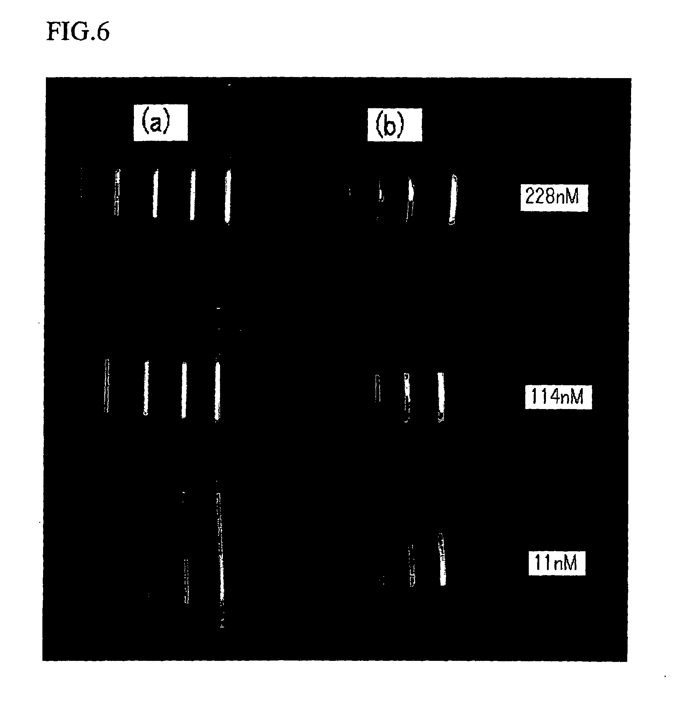

The results are indicated in Figure 6. In Figure 6, (a) is the results when using cloudy semitransparent nylon thread; and (b) is when using transparent nylon thread.

As indicated in Figure 6, it could be clarified that the cloudy semitransparent nylon thread had greater background, and therefore, it is preferable to use the transparent nylon thread which has a smaller background, when the concentration of the labeled nucleic acid is low. It was also clarified that either the cloudy semitransparent nylon thread or the transparent nylon thread could be used when the concentration of the labeled nucleic acid is high.

### INDUSTRIAL APPLICABILITY

The present invention provides a carrier for fixing material containing amino groups, on the surface of which a material containing an amino group can be immobilized via covalent bonding (amide bonding); a carrier on the surface of which a material containing an amino group has been immobilized via covalent bonding (amide bonding); and a method of immobilizing a material containing an amino groups on the surface of a carrier via covalent bonding (amide bonding).

According to the carrier and the immobilization method of the present invention, it is possible to easily and efficiently immobilize a material containing an amino group on the surface of the carrier by using a carboxyl group or an amino group, or a derivative group thereof formed by cleaving an amide bond in the main chain of a polyamide. Specifically, it is possible to immobilize more materials containing an amino group onto the surface of the carrier by converting an amino group formed by cleaving an amide bond in the main chain of a polyamide, into a carboxyl group or a derivative group thereof.

In the carrier on the surface of which a material containing an amino group has been immobilized of the present invention, there is no concern that the material will fall off of the surface of the carrier because the immobilized material has been immobilized on the surface of the carrier via amide bonding (covalent bonding). Consequently, by using the carrier on the surface of which a material containing an amino group has been immobilized of the present invention, it is possible to easily and efficiently detect and isolate a material containing an amino group such as nucleic acid and protein.

## Claims

1. A carrier for fixing a material containing an amino group, which has a shape to be wound on a spindle member, and has a carboxyl group or an amino group, or a derivative group thereof formed by cleaving an amide bond in the main chain of a polyamide on its surface.

2. The carrier claimed in Claim 1, obtained by hydrolyzing a crude material member which has a shape to be wound on a spindle member, and has a polyamide exposed on its surface, to cleave an amide bond in the main chain of the polyamide.

3. The carrier claimed in Claim 2, wherein the crude material member is a polyamide fiber or an aggregate thereof.

4. The carrier claimed in any of Claims 1 to 3, wherein the shape of the carrier is filament-like, string-like or tape-like.

5. The carrier claimed in any of Claims 1 to 3, wherein the amino group formed by cleaving the amide bond in the main chain of the polyamide is converted to a carboxyl group or a derivative group thereof by reacting the amino group with a cyclic acid anhydride.

6. A carrier on the surface of which a material containing an amino group has been immobilized, obtained by reacting a carboxyl group or a derivative group thereof on the surface of the carrier claimed in any of Claims 1 to 3 with the amino group of the 'material, to immobilize the material on the surface of the carrier via an amide bond.

7. A method of immobilizing a material containing an amino group on a carrier which has a shape to be wound on a spindle member, and has a polyamide exposed on its surface;
the method comprising:
(a) a process to produce a carboxyl group or an amino group, or a derivative group thereof on the surface of the carrier by hydrolyzing the carrier to cleave an amide bond in the main chain of the polyamide; and
(b) a process to immobilize the material on the surface of the carrier via an amide bond by reacting the carboxyl group or the derivative group thereof on the surface of the carrier with the amino group of the material.

8. The method claimed in Claim 7, wherein the amino group on the surface of the carrier is converted to a carboxyl group or a derivative group thereof by reacting the amino group with a cyclic acid anhydride, prior to the process (b).
